# EUROPÄISCHE PATENTSCHRIFT

(11) **EP 0 920 535 B1**
(45) Veröffentlichungstag und Bekanntmachung des Hinweises auf die Patenterteilung: **14.11.2007**
(21) Anmeldenummer: 97941854.8
(22) Anmeldetag: 26.08.1997
(51) Int. Cl.: C12Q 1/68

(54) **VERFAHREN ZUM NACHWEIS KLINISCH RELEVANTER VERÄNDERUNGEN DER DNS-SEQUENZ DES KI-RAS-ONKOGENS, SEINE VERWENDUNG UND TESTKIT ZUR FRÜHERKENNUNG VON TUMOREN**
METHOD TO DETECT CLINICALLY RELEVANT MUTATIONS OF THE DNA SEQUENCE OF KI -RAS ONCOGENE, ITS USE AND A TESTKIT FOR EARLY DIAGNOSIS OF TUMOURS
PROCEDE DE DETECTION DE MODIFICATIONS CLINIQUEMENT SIGNIFICATIVES DE LA SEQUENCE ADN DE L'ONCOGENE KI-RAS, SON UTILISATION ET KIT D'ESSAI POUR LE DIAGNOSTIC PRECOCE DE TUMEURS

(30) Priorität: 26.08.1996 DE 19635609
(43) Veröffentlichungstag der Anmeldung: 09.06.1999
(73) Patentinhaber: Invitek Gesellschaft für Biotechnik & Biodesign mbH., 13125 Berlin (DE)
(72) Erfinder: HILLEBRAND, Timo, D-12619 Berlin (DE); BERNDT, Hans-Christoph, D-13187 Berlin (DE); BENDZKO, Peter, D-12623 Berlin (DE)
(74) Vertreter: Baumbach, Friedrich
(86) Internationale Anmeldenummer: PCT/DE1997/001894
(87) Internationale Veröffentlichungsnummer: WO 1998/008971

(56) Entgegenhaltungen:
- EP-A- 0 535 376
- WO-A-93/20235
- WO-A-95/34569
- DE-A- 19 530 132
- US-A- 4 902 783
- CALDAS ET AL.: "Detection of K-ras mutations in the stool of patients with pancreatic adenocarcinoma and pancreatic ductal hyperplasia" CANCER RESEARCH, Bd. 54, Juli 1994, Seiten 3568-3573, XP002050203 in der Anmeldung erwähnt
- WILDE ET AL.: "Removal of inhibitory substances from human fecal specimens for detection of group A rotaviruses by reverse transvriptase and PCR" JOURNAL OF CLINICAL MICROBIOLOGY, Bd. 28, 1990, WASHINGTON, US, Seiten 1300-1307, XP000406095
- SIDRANSKI ET AL.: "Identification of ras oncogene mutations in the stool of patients with curable colorectal tumors" SCIENCE, Bd. 256, April 1992, LANCASTER, PA US, Seiten 102-105, XP000605488 in der Anmeldung erwähnt

## Beschreibung

Die Erfindung betrifft ein Verfahren zum Nachweis klinisch relevanter Veränderungen der DNS-Sequenz des Ki-ras-Onkogens in Stuhl-DNS, seine Verwendung sowie einen Testkit zur Früherkennung von Tumoren, insbesondere von Tumoren des Pankreas und Dickdarms.

Dickdarm- und Pankreastumore zählen zu den weltweit häufigsten Krebserkrankungen und stehen an dritter bzw. vierter Stelle in der Mortalitätsstatistik von Malignomen. Die Problematik dieser Erkrankungen tritt besonders deutlich beim Pankreaskarzinom zutage. Bedingt durch einen über lange Zeit symptomlosen Verlauf werden Pankreaskarzinome so spät diagnostiziert, daß die durchschnittliche 5-Jahre-Überlebensrate der Patienten trotz ausgefeilter chirurgischer Techniken 2% nicht übersteigt.

Gegenwärtig gibt es keine befriedigenden Laborparameter für die Früherkennung von Pankreastumoren. Zur Diagnostik kolorektaler Karzinome wird der Hämokkult-Test eingesetzt, bei welchem okkultes Blut im Stuhl nachgewiesen wird. Die analytische Aussagekraft dieses Testes ist unbefriedigend, weil okkultes Blut im Stuhl auch bei nichtmalignen Erkrankungen wie Hämorrhoiden auftritt, und weil positive Befunde darüber hinaus auch durch eine Vielzahl interferierender Stuhlkomponenten wie Peroxydasen und Katalasen verursacht sein können. Andererseits ist bekannt, daß Tumoren mit einem Durchmesser von weniger als 2 cm nicht genügend Blut abgeben, um im Hämokkult-Testverfahrens aufzufallen.
So liegt selbst bei fortgeschrittenen Dickdarmtumoren die diagnostische Sensitivität des Hämokkult-Testes bei nur 50-70%.

Eine mögliche Alternative für die Früherkennung von Pankreas-und Dickdarmtumoren bietet der molekularbiologische Nachweis von relevanten Mutationen in Onkogenen, vorzugsweise im Protoonkogen Ki-ras. So konnte nachgewiesen werden, daß Mutationen im Ki-ras Protoonkogen bei 75-90% von Pankreaskarzinomem (Almoguera, S., Shiate, D., Forrester, K., Martin, J., Arnheim, N., Perucho, M. (1988) Cell 53, 549-554) sowie bei wenigstens 50% kolorektaler Karzinomen (Forrester, K., Alomugera, C., Han, K., Grizzle, W.E., Perrucho, M. (1987) Nature 327, 298-303) vorkommen.

Die Frequenz beim Pankreaskarzinom ist bisher die höchste, welche für ein Onkogen bei einem spezifischen Tumor gefunden wurde. -
Darüber hinaus sind die relevanten Mutationen auf die Kodons von drei Aminosäuren beschränkt; beim Pankreaskarzinom nur auf eine Aminosäure. Dieser Umstand kann den Mutationsnachweis beträchtlich vereinfachen.

Sowohl bei Dickdarm- als auch bei Pankreastumoren gibt es die Möglichkeit eines nicht invasiven Nachweises des Onkogenstatus im Stuhl. Ein solcher Nachweis wurde erstmals 1992 von Sidransky und Mitarbeitern gezeigt (Sidransky, D., Tokino, T., Hamilton, S.R., Kinler, K.W., Levin, B., Vogelstein, B. (1992); Science 256, 102-105). Er gelingt, weil zum einen eine genügende Zahl von Pankreas-und Darmepithelzellen in den Stuhl gelangen, wobei Tumorzellen unter den Bedingungen im Stuhl möglicherweise stabiler sind als normale Epithelien. Zum zweiten besitzen Bakterien keine Gene für Ki-ras, so daß die Diagnostik nicht durch die Interferenz von Darmflora und Epithelzellen beeinträchtigt wird. Über die Detektion von aktiviertem Ki-ras lassen sich Adenome bis zu einer Größe von 1 cm³ diagnostizieren.

Trotz dieser günstigen Vorbedingungen für eine labordiagnostische Anwendung findet sich bis heute kein Nachweisverfahren für Ki-ras Mutationen in Stuhl-DNS, das routinemäßig eingesetzt werden kann.

Das Hauptproblem hierbei liegt in der enormen Schwierigkeit begründet, DNS von ausreichender Qualität mit einem vertretbaren Aufwand aus Stuhlproben zu isolieren. Die zur Zeit am häufigsten praktizierte Extraktionsmethode beinhaltet eine Reihe von mehrstündigen Reinigungsschritten und erstreckt sich in seiner Gesamtdurchführung auf mindestens eine Arbeitswoche.

Auch weitere Verbesserungen dieser Methode durch Caldas und Mitarbeiter erfordern auch einen mehrtägigen Durchführungsaufwand (Caldas, C., Hahn, S.A., Hruban, R.H., Redston, M.S., Yeo, C.J., Kern, S.E. (1994); Cancer Res. 54, 3568-3573).

Stuhl ist ein komplexes Gemisch aus abgeschilferten Zellen, Mikroorganismen, unverdauten Nahrungsbestandteilen, Schleim- und Farbstoffen sowie anderen löslichen und unlöslichen Komponenten des Gastrointestinaltraktes. Eine solche komplexe Zusammensetzung bedingt das Vorhandensein einer Vielzahl von inhibitorischen Stoffen, welche sowohl als kontaminierende Bestandteile der isolierten DNS-Lösung wie auch direkt in die DNS intercalieren oder an der DNS gebunden sind und aus diesem Grunde eine Verwendung der isolierten DNS für weitere Untersuchungen verhindern.

Der Erfindung lag deshalb die Aufgabe zugrunde, ein routinemäßig einsetzbares Nachweisverfahren für ein entsprechendes Gendiagnostiksystem zu entwickeln, wobei einer geeigneten DNS-Extraktionsmethode eine Schlüsselfunktion für die Etablierung eines routinetauglichen und letzlich auch automatisierbaren Ki-ras Tests zukommt, sowie einen darauf aufbauenden Testkit bereitzustellen.

Die Aufgabe wird gemäß der Ansprüche 1 bis 13 realisiert. Sie wird erfindungsgemäß dadurch gelöst, daß über multiple Reinigungsschritte aus Stuhlproben genomische DNS extrahiert wird, wobei alle inhibitorischen Stoffe hochselektiv eliminiert werden, so daß die isolierte genomische DNS problemlos für weitere Anwendungen verfügbar ist.

Stuhlproben werden erfindungsgemäß in einem ersten Schritt mit Materialien, die Adsorptionseigenschaften aufweisen, zur Entfernung inhibitorischer Stoffe inkubiert. Die in den Proben enthaltenen Zellen werden mit einem Puffer, der chaotrope Salze enthält, lysiert.
Die nachfolgend an ein mineralisches Trägermaterial gebundene genomische DNS wird in einem weiteren Waschprozeß weiter aufgereinigt und danach mittels eines Puffers geringer Ionenstärke vom Trägermaterial abgelöst. In einem abschließenden Aufreinigungsschritt werden dann auch die in die oder an die DNS gebundenen Stoffe (Inhibitoren) entfernt. Gerade diese in oder an DNS gebundenen Stoffe stellen potente Inhibitoren dar. Diese werden durch Inkubation der bereits isolierten DNS mit einem Puffer mit einer Ionenstärke > 4M, welcher ein chaotropes Salz enthält, so z.B. aus Natriumjodid von der DNS verdrängt. Eine anschließende Zugabe des Trägermaterials zur erneuten Bindung der DNS, nachfolgendes Waschen und Elution der DNS bilden den Abschluß des Reinigungsverfahrens. Die auf diese Weise isolierte DNS aus den Proben steht nun weiteren molekularbiologischdiagnostischen Techniken zur Verfügung.

Im einzelnen werden erfindungsgemäß folgende Reinigungsschritte durchgeführt:
a) Inkubation mit einer Lösung aus Aktivkohle, zur Entfernung inhibitorischer Stoffe bei Verwendung einer Stuhlprobe;
b) Lyse der in den Materialproben enthaltenen Zellen mit einem Puffer, der chaotrope Salze enthält, wie z. B. Guanidinisothiocyanat, Guanidinhydrochlorid, Lithiumchlorid oder Lithiumchlorid/Harnstoff-Gemische mit Ionenstärken >4M;
c) Inkubation des Lysates mit einem mineralischen Trägermaterial zur Bindung der DNS; vorzugsweise handelt es sich bei dem Trägermaterial um hochdisperse, nichtporöse SiO₂-Partikeln mit einer Korngröße von 7 nm - 1 µm, vorzugsweise 40 nm, bei einer spezifischen Oberfläche von 10-300 m²/g, vorzugsweise 50 m²/g,
d) Abtrennung der Trägermaterials vom Lysat durch Zentrifugation,
e) Waschen der am Trägermaterial gebundenen DNS, vorzugsweise einem Waschpuffer bestehend aus 50 mM NaCl, 10 mM Tris HCl und 1 mM EDTA sowie 70%v/v Ethanol, und Ablösen der DNS durch Inkubation des Trägermaterials mit einem Puffer geringer Ionenstärke, vorzugsweise 10 mM Tris HCl; 0,1 mM EDTA bei einer Temperatur von 48-65°C,
f) Inkubation der abgelösten genomischen DNS mit einem Puffer, welcher ein chaotropes Salz enthält, vorzugsweise aus Natriumjodid, Natriumperchlorat oder Kaliumjodid mit Ionenstärken >4M bei kontinuierlichem Schütteln, vorzugsweise mit 5M Natriumjodid, zur Entfernung an die DNS gebundenen Substanzen aus der genomischen DNS,
g) Inkubation der Pufferlösung mit der genomischen DNS erneut mit einem mineralischen Trägermaterial, vorzugsweise aus hochdispersen, nichtporösen SiO₂-Partikeln mit einer Korngröße von 7 nm - 1 µm, vorzugsweise 40 nm, bei einer spezifischen Oberfläche von 10-300 m²/g, vorzugsweise 50 m²/g,
h) Abtrennen der Lösung vom mineralischen Trägermaterial durch Zentrifugation,
i) Waschen des Trägermaterials mit der gebundenen DNS, vorzugsweise mit einem Puffer bestehend aus 50 mM NaCl, 10 mM Tris HCl und 1 mM EDTA sowie 70%v/v Ethanol, und wiederum Ablösen der gebundenen genomischen DNS mit einem Puffer geringer Ionenstärke, bevorzugt 10 mM Tris HCl, 0,1 mM EDTA bei einer Temperatur von 48-65°C.

Das Extraktionsverfahren wird gemäß der Erfindung bevorzugt als batch- Verfahren oder als säulenchromatographisches Verfahren oder als chromatographisches Verfahren in Mikrotiterplattenformat durchgeführt.

Das gesamte Extraktionsverfahren benötigt weniger als 2h für mindestens 10 unterschiedliche Proben und löst damit erstmalig in idealster Weise das Problem der Extraktion genomischer DNS aus Stuhlproben und anderen Materialien. Die Erfindung erlaubt die Isolierung von DNS aus abgeschilferten Zellen aus Stuhlproben in extrem kurzer Zeit, wobei überraschenderweise durch den Einsatz einer Lösung aus Aktivkohle im erfinderischen Reinigungsverfahren inhibitorische Stoffe, welche nicht direkt an die genomische DNS gebunden sind, eliminiert werden konnten.

Der erfindungsgemäße Nachweis klinisch relevanter Veränderungen der DNS Sequenz des Ki-ras Gens erfolgt anschließend über basenkomplementäre Hybridisierungsreaktionen nach an sich bekannten Techniken mit mindestens sechs verschiedenen Oligonukleotiden mit definierter Komplementarität zu möglichen mutativ veränderten Sequenzabschnitten des Ki-ras Gens.

Nach erfolgter Extraktion der genomischen DNS aus der klinischen Probe schließt sich die Erzeugung der zu analysierenden Ki-ras-Sequenz an.

Die Oligonukleotide und die nachzuweisenden sequenzveränderten Abschnitte des Ki-ras Gens werden inkubiert, wobei entweder die Oligonukleotide oder die nachzuweisenden sequenzveränderten Abschnitte des Ki-ras Gens markiert sind und der Nachweis des Hybridisierungsergebnisses über die Markierung detektiert wird oder andere an sich bekannte Nachweisverfahren für Duplex-DNS zum Nachweis des Hybridisierungsergebnisses verwendet werden.

Die Oligonukleotide und/oder die sequenzveränderten Abschnitte des Ki-ras Gens oder hybridisierte DNS befinden sich dazu an einer festen Phase, die vorzugsweise eine Mikrotiterplatte, ein Lichtwellenleiter oder ein Siliciumchip ist, besonders bevorzugt eine Mikrotestplatte.

Die Amplifikationsreaktion erfolgt mit einem Primerpaar, wobei einer der Primer an seiner 5'-Position mit einer Markierung, vorzugsweise einer Biotin-Markierung, gekoppelt ist. Das nach einer Vervielfälltigungsreaktion generierte DNS-Fragment ist dadurch ebenfalls markiert. Diese Markierung ermöglicht nachfolgend die Kopplung des amplifizierte Ki-ras-Fragmentes in einer bevorzugten Ausführungsvariante an die Oberfläche einer Streptavidin beschichteten festen Phase, vorzugsweise an die Oberfläche einer Mikrotestplatte über eine Streptavidin-Biotin-Brücke.

Das an der Oberfläche gebundene doppelsträngige Fragment wird unter Zugabe einer an sich bekannten Denaturierungslösung, wie z.B. NaOH, in einen Einzelstrang überführt. Auf der Oberfläche der Mikrotestplatte verbleibt nach Absaugen der Denaturierungslösung nur der über die Biotin-Streptavidin-Brücke fixierte DNS-Strang. Dieser ist somit der Analyt und entspricht dem auf das Vorhandensein von Einzelbasenmutationen zu untersuchenden Ki-ras-Abschnitt. Der plattenfixierte Ki-ras-Einzelstrang wird in nachfolgenden Schritten mit allelspezifischen Oligonukleotiden (Hybridisierungssonden) unter einem Standard-Hybridisierungspuffer inkubiert. Die Oligonukleotide entsprechen dabei jeweils einer möglichen Ki-ras Punktmutation bzw. der Ki-ras Wildtypsequenz.
Die Auswahl der Oligonukleotide erfolgte erfindungsgemäß unter dem Aspekt, die Hybridisierungreaktionen für jede der verschiedenen allelspezifischen Oligonukleotide unter absolut identischen Reaktionsparametern durchzuführen, um Mutationstests simultan für unterschiedliche Punktmutationen auf einer Mikrotestplatte zu realisieren.

Die 6 Oligonukleotide weisen vorzugsweise die Sequenzen
5'-GTT-GGA-GCT-CGT-GGC-GTA-3'
5'-GTT-GGA-GCT-TGT-GGC-GTA-3'
5'-GTT-GGA-GCT-AGT-GGC-GTA-3'
5'-GTT-GGA-GCT-GCT-GGC-GTA-3'
5'-GTT-GGA-GCT-GAT-GGC-GTA-3'
5'-GTT-GGA-GCT-GTT-GGC-GTA-3'

Der entscheidende Schritt für den Nachweis einer Einzelbasenmutation besteht in hochoptimierten Reaktionsbedingungen für essentielle Waschschritte nach der erfolgten Hybridisierungsreaktion.
Gemäß der Erfindung erfolgen ein bis fünf, vorzugsweise drei, Waschschritte nach einer 15 bis 45-minütigen, vorzugsweise 30 minütigen, Inkubationszeit bei 38 - 45 °C, vorzugsweise 42 °C, bei jeweils 5 bis 15 Minuten, vorzugsweise 10 Minuten, mit einer Waschlösung aus SDS und SSC bei einer Temperatur von 45 bis 55 °C, vorzugsweise 0,03 % SDS und 0,03 % SSC bei 50 °C und 10 Minuten.
Entscheidend dabei ist, daß nur bei vollständiger Basenkomplementarität zwischen Ki-ras-Target und dem jeweiligen Hybridisierungsoligonukleotid ein Hybridisationsprodukt erhalten bleibt, jede Baseninkomplementarität aber zur selektiven Entfernung der Oligonukleotidsonde führt.

Diese Bedingungen konnten im erfinderischen Verfahren in idealster Weise gelöst werden. Die optimierten Bedingungen der Waschschritte erlauben dabei zusätzlich die simultane Testdurchführung zum Nachweis von sechs verschiedenen Ki-ras-Mutationen auf einer Testplatte.

Der Nachweis des Hybridisierungsergebnisses erfolgt über eine dem Fachmann an sich bekannten indirekt enzymatischen kolorimetrischen Detektion. Diese basiert darauf, daß die Hybridisierungsoligonukleotide mit einer Standardmarkierung z.B. Digoxigenin oder FITC versehen sind, gegen welche enzymkonjugierte Antikörper eingesetzt werden. Nach Zugabe einer Substratlösung wird letztlich via Durchlichtmessung in einem Mikrotestplatten-Reader die Intensität der Farbreaktion bestimmt. Sie ist das Parameter für die Befundung des Hybridisierungsergebnisses. Mitgeführte Standard-DNS Proben für eine Ki-ras Mutation bzw. für Ki-ras Wildtyp werden als Bezugsgrößen für die Bewertung der Probenanalysen herangezogen.

Das Nachweisverfahren wird bevorzugt in Mikrotiterplatten durchgeführt und hat somit den Vorteil, daß die Möglichkeit der Analyse einer Vielzahl unterschiedlicher Proben besteht, wodurch eine Automatisierung des Verfahrens ermöglicht wird.

Bisher bekannte Nachweisverfahren von Mutationen über Hybridisierungsreaktionen werden nur mittels bekannter molekularbiologischer Techniken, wie z.B. Dot-Blot-Hybridisierungen, an Filtermembranen durchgeführt, bei welchen der Nachweis der Hybridisierungsreaktion über die Detektion einer radioaktiven Markierung erfolgt. Ein solches Verfahren ist extrem zeitaufwending und durch die Verwendung radioaktiver Nachweisverfahren zudem hochgefährlich, ebenso ist eine Automatisierbarkeit nicht möglich.

Das erfinderische Verfahren benötigt einschließlich der DNS-Extraktion aus den Proben ca. 8h. Dies ist ein Zeitaufwand, welcher es zum ersten Mal ermöglicht, den Ki-ras Status in einer routinemäßigen Labordiagnotik als den wohl wichtigsten klinischen Parameter für die Früherkennunng maligner Veränderungen, vorzugsweise von Kolon und Pankreas zu nutzen.

Eine solche Diagnostik wird wesentlich dazu beitragen, die Mortalitätsraten dieser Erkrankungen senken zu helfen.

Die Erfindung betrifft außerdem einen Testkit zum Nachweis von Mutationen im Ki-ras-Gen. Diese ist durch einen modularen Aufbau charakterisiert und besteht aus:
1. DNS-Extraktionssystem,
   a) eine Lösung aus Aktivkohle, zur Entfernung inhibitorischer Stoffe bei Verwendung einer Stuhlprobe,
   b) zur Lyse der in den Materialproben enthaltenen Zellen einen Puffer, der chaotrope Salze enthält, wie z. B. Guanidinisothiocyanat, Guanidinhydrochlorid, Lithiumchlorid oder Lithiumchlorid/Harnstoff-Gemische mit Ionenstärken >4M; besonders bevorzugt Guanidinisothiocyanat; DTT, Natriumcitrat,
   c) zur Inkubation des Lysates einen mineralischen Träger Bindung der DNS; vorzugsweise hochdisperse, nichtporöse SiO₂-Partikel mit einer Korngröße von 7 nm - 1 µm, vorzugsweise 40 nm, bei einer spezifischen Oberfläche von 10-300 m²/g, vorzugsweise 50 m²/g,
   d) einen Waschpuffer bestehend aus 50 mM NaCl, 10 mM Tris HCl und 1 mM EDTA sowie 70%v/v Ethanol, und Ablösen der DNS durch Inkubation des Trägermaterials mit einem Puffer geringer Ionenstärke, vorzugsweise 10 mM Tris HC1;0,1 mM EDTA,
   e) zur Inkubation der abgelösten genomischen DNS einen weiteren Puffer, welcher ein chaotropes Salz enthält, vorzugsweise aus Natriumjodid, Natriumperchlorat oder Kaliumjodid mit Ionenstärken >4M, besonders bevorzugt 5 M Natriumjodid,
   i) zum Waschen des Trägermaterials mit der gebundenen DNS, vorzugsweise einen Puffer bestehend aus 50 mM NaCl, 10 mM Tris HCl und 1 mM EDTA sowie 70%v/v Ethanol,
2. Primermixen zur selektiven Amplifikation der zu untersuchenden Ki-ras-Targetsequenz,
3. Kontrollzellinien-DNA zur Amplifikation von Wildtyp bzw. Mutationstyp einer Ki-ras-Zellinie,
4. 6 Oligonukleotidsonden mit den Sequenzen
   5'-GTT-GGA-GCT-CGT-GGC-GTA-3'
   5'-GTT-GGA-GCT-TGT-GGC-GTA-3'
   5'-GTT-GGA-GCT-AGT-GGC-GTA-3'
   5'-GTT-GGA-GCT-GCT-GGC-GTA-3'
   5'-GTT-GGA-GCT-GAT-GGC-GTA-3'
   5'-GTT-GGA-GCT-GTT-GGC-GTA-3' ,
5. eine feste Phase, vorzugsweise eine Mikrotestplatte einschließlich für den Platten-Assay notwendiger Reagenzien wie oben beschrieben.

Das System wird vorzugsweise für die Untersuchung von Stuhl-DNS zur Früherkennung von gastrointestinalen Tumoren, speziell für die Früherkennung präneoplastischer kolorektaler proliferativer Erkrankungen eingesetzt.

Durch die Universalität des DNS-Extraktionsverfahrens und über den modularen Aufbau des Testkits besteht aber auch die Möglichkeit, andere klinisch relevante Ausgangsmaterialien auf Ki-ras-Mutationen zu untersuchen. Dabei bleibt das Nachweissystem immer konstant.

Die eigenen Untersuchungsergebnisse erbrachten so auch den Nachweis von Ki-ras Mutationen nach erfolgter DNS-Extraktion aus Pankreassekreten (ercp-Proben). Es besteht damit erstmalig die Möglichkeit des Auffindens von Risikopatienten für Pankreastumoren nach Untersuchung dieses Probenmaterials. Dem Fachmann ist bekannt, daß endzündliche Prozesse des Pankreas (akute oder chronische Pankreatiden) häufig in maligne Phänotypen entarten können. Ein vorhandener Pankreastumor ist zum Zeitpunkt seines Nachweises aber nicht mehr kurativ behandelbar. Der Nachweis von mutativen Veränderungen im Ki-ras-Gen kann somit als entscheidender Parameter für eine diesbezügliche Prognose herangezogen werden.

Das erfinderische Verfahren eignet sich darüber hinaus auch in idealer Weise zur Untersuchung von Biopsieproben aus gastrointestinalen Polypen nach endoskopischer Entnahme. Auch bei diesen Proben kann über die Detektion von Ki-ras Mutationen frühzeitig eine potentiell bestehende mögliche maligne Veränderungen diagnostiziert und können Patienten als Risikopatienten eingestuft werden. Über eine rechtzeitig erfolgende chirurgische Entfernung Ki-ras-mutierter Polypen kann so die Entstehung eines Tumors unterbunden werden. Bis heute findet eine solche Diagnostik auf Grund des Fehlens eines routinetauglichen Nachweissystems nicht statt.

Gemäß der Erfindung werden zur Untersuchung der Ki-ras-Targetsequenz mindestens 6 verschiedenen Oligonukleotide, die definierte Komplementarität zu bekannten mutativ veränderten Sequenzabschnitten des Ki-ras Gens aufweisen, eingesetzt. Damit besteht die Möglichkeit, mehr als 80% aller in Zusammenhang mit einem malignen Phänotyp stehenden Ki-ras Mutatione eindeutig zu detektieren.

Anschließend soll die Erfindung an einem Beispiel näher erläutert werden.

### Ausführungsbeispiel:

### Nachweis von Ki-ras-Punktmutatioen in Stuhl-DNS

Überführen von ca. 300-500 mg einer Stuhlprobe in ein 2.0 ml Eppendorf-Reaktionsgefäß. Zugabe von 350 µl einer Waschlösung (NaCl, Tris; EDTA) und vortexen.

Zentrifugation für 2 min bei 14000 rpm und Überführen des Überstandes in ein neues 2.0 ml Eppendorf-Reaktionsgefäß.

Zugabe von 250 µl einer Lösung aus Aktivkohle; 10 min Schütteln der Probe. Zentrifugation für 2 min bei 14000 rpm und Überführen des Überstandes in ein neues 1,5 ml Eppendorf-Reaktionsgefäß. Zentrifugation für 1 min bei 14000 rpm und Überführen des Überstandes in ein neues 2,0 ml Eppendorf-Reaktionsgefäß.

Zugabe von 1 ml Puffer (Guanidinisothiocyanat; DTT, Natriumcitrat) zur Zell-Lyse und Inkubation für 30 min bei Raumtemperatur.

Zugabe von 20 µl einer mineralischen Trägersuspension aus hochdispersen, nichtporösen SiO₂- Partikeln mit einer Korngröße von ca. 40 nm, bei einer spezifischen Oberfläche von ca. 50 m²/g, und Inkubation für 5 min bei Raumtemperatur. Zentrifugation für 1 s bei 10000 rpm und sorgfältiges Entfernen des Überstandes.

Zugabe von 1 ml Waschpuffer (NaCl, Tris, EDTA, Ethanol) und Resuspendieren des Pellets. Zentrifugation für 1 s bei 10000 rpm und sorfältiges Entfernen des Überstandes. Zweimaliges Wiederholen des Waschschrittes. Kurze Inkubation des geöffneten Reaktionsgefäßes bei 60°C bis zur vollständigen Entfernung des Ethanols.

Zugabe von 100 µl eines Elutionsmittels (10mM Tris-HCl; 0.1mM EDTA), Resuspendieren des Pellets und Inkubation des Reaktionsgefäßes für 5 min bei 60°C. Zentrifugation für 2 min bei 14000 rpm und Überführen des Überstandes in ein neues 1,5 ml Eppendorf-Reaktionsgefäß. Mischen der isolierten DNS mit einer Lösung aus Natriumjodid unter leichtem Schütteln für 5 min.

Erneute Zugabe von 15 µl der mineralischen Trägersuspension zur Pufferlösung mit der genomischen DNS und Inkubation für 5 min auf Eis. Zentrifugation für 1 s bei 10000 rpm und sorgfältiges Entfernen des Überstandes.

Zugabe von 1 ml Waschpuffer und Resuspendieren des Pellets. Zentrifugation für 1 s bei 10000 rpm und sorgfältiges Entfernen des Überstandes. Zweimaliges Wiederholen des Waschschrittes. Kurze Inkubation des geöffneten Reaktionsgefäßes bei 60°C bis zur vollständigen Entfernung des Ethanols. Zugabe von 50 µl Elutionspuffer (10 mM Tris-HCl; 0.1 mM EDTA), Resuspendieren des Pellets und Inkubation des Reaktionsgefäßes für 5 min bei 60°C. Zentrifugation für 2 min bei 14000 rpm und Überführen des Überstandes in ein neues 1,5 ml Eppendorf-Reaktionsgefäß.

Nach der erfolgten DNS-Extraktion werden 1-10µl der DNS für die enzymatische Vervielfältigung der zu untersuchenden spezifischen Targetsequenz des Ki-ras Gens mittels Polymerase Kettenreaktion (PCR) eingesetzt.
In einer ersten PCR-Reaktion erfolgt dabei die Amplifikation mit einem Primermix, bei welchem während der Amplifikationsreaktion von Ki-ras-Wildtyp-DNA eine Restriktionschnittstelle generiert wird. In alle amplifizierten DNA-Fragmente welche nicht wildtypkonform sind, erfolgt dagegen kein Einbau dieser Schnittstelle.

Nach der erfolgten Amplifizierungsreaktion wird unter Einsatz eines Restriktionsenzyms der Ki-ras-Wildtyp zerschnitten.

In einer zweiten Amplifikationsreaktion werden 1-2 µl des Restriktionsansatzes als Template DNS eingesetzt. Dabei erfolgt nunmehr die selektive Anreicherung der ungeschnittenen mutierten Ki-ras-Fragmente. Diese Amplifikationsreaktion erfolgt mit einem Primerpaar, bei welchem ein Primer an seiner 5'-position Biotin-markiert ist.

Nach erfolgter Amplifikationsreaktion werden 1-5 µl des Amplifikationsproduktes in 420 µl eines Bindungspuffers (Tris; EDTA, NaCl) aufgenommen.

Jeweils 50 µl dieses Ansatzes werden auf 7 Löcher (Wells) einer Mikrotestplatte verbracht. Diese Wells dienen nachfolgend der Analyse des Ki-ras-Fragmentes auf mutative Veränderungen.

Nach einer kurzen Inkubation wird die Bindungspufferlösung abgesaugt, 50 µl einer Denaturierungslösung (NaOH) zugegeben und unter leichtem Schütteln inkubiert. Hierbei erfolgt nun die Generierung eines DNA-Einzelstranges, gegen welchen nachfolgend eine jeweils sequenzspezifische Hybridisierung durchgeführt wird. Nach Entfernung des nicht an der Plattenoberfläche fixierten zweiten DNA-Stranges mit einem Waschpuffer (Tris, EDTA, NaC1) wird jedes der 7 Wells der Mikrotestplatte mit einer der spezifischen z.B. Digoxygenin oder FITC markierten Oligonukleotidsonden unter einem Standard-Hybridisierungspuffer benetzt.
Nach einer 30 minütigen Inkubationszeit bei 42°C erfolgt die Hybridisierung der jeweiligen Sonden mit dem an der Plattenoberfläche gebundenen Ki-ras-Fragment.

Die Spezifität des Nachweises wird durch nachfolgend durchzuführende Waschschritte unter hochstringenten Konditionen erreicht. Diese Waschschritte erfolgen mit einer Lösung aus 0,03% SDS; 0,03xSSC bei 50°C für 3x10min. Unter diesen Konditionen werden alle Sonden, die keine vollständige Basenkomplementarität zur Ki-ras-Targetsequenz aufweisen, entfernt.
In nachfolgenden Schritten erfolgt nach an sich bekannten klassischen kolorimetrischen Verfahren die Detektion des Hybridisierungsergebnisses unter Verwendung eines Mikrotestplatten-Readers. Die gemessene Signalstärke dient dabei als das Auswertungskriterium für den Nachweis eines Hybridisierungsproduktes, und somit als Nachweis auf das Vorliegen einer Mutation oder von Wildtyp Ki-ras.

## Patentansprüche

1. Verfahren zum Nachweis klinisch relevanter Veränderungen der DNS-Sequenz des Ki-ras Onkogens in DNS, wobei Stuhlproben als Ausgangsmaterial dienen,
**gekennzeichnet durch**
- Extraktion genomischer DNS aus Stuhlproben über multiple Reinigungsschritte zur Eliminierung inhibitorischer Stoffe, wobei die Extraktion der genomischen DNS erfolgt **durch**:
- Entfernung inhibitorischer Stoffe **durch** Inkubation mit einer Lösung aus Aktivkohle,
- Lyse der in der Stuhlprobe enthaltenen Zellen mit einem Puffer, der die chaotropen Salze Guanidinisothiocyanat, Guanidinhydrochlorid, Lithiumchlorid oder Lithiumchlorid/Harnstoff-Gemische enthält;
- Inkubation des Lysates mit einem mineralischen Trägermaterial aus nichtporösen SiO₂- Partikeln zur Bindung der DNS;
- Abtrennung des Trägermaterials vom Lysat **durch** Zentrifugation;
- Waschen der am Trägermaterial gebundenen DNS mit einem Gemisch aus 50 mM NaCl, 10 mM Tris HCl und 1 mM EDTA sowie 70%v/v Ethanol und Elution der DNS **durch** Inkubation des Trägermaterials mit einem Puffer geringer Ionenstärke aus 10 mM Tris HCl, 0,1 mM EDTA bei einer Temperatur von 48-65°C;
- Inkubation der abgelösten genomischen DNS mit einem Puffer aus Natriumjodid, Natriumperchlorat oder Kaliumjodid zur Entfernung von an die DNS gebundenen Substanzen aus der genomischen DNS;
- erneute Inkubation der Pufferlösung mit der genomischen DNS mit einem mineralischen Trägermaterial;
- Abtrennen der Lösung vom mineralischen Trägermaterial **durch** Zentrifugation;
- Waschen des Trägermaterials mit der gebundenen DNS und erneut Elution der gebundenen genomischen DNS mit einem Puffer geringer Ionenstärke
und
- basenkomplementäre Hybridisierungsreaktion **durch** Zugabe von den folgenden sechs Oligonukleotiden mit definierter Komplementarität zu veränderten Sequenzabschnitten des Ki-ras Gens:
5'-GTT-GGA-GCT-CGT-GGC-GTA-3'
5'-GTT-GGA-GCT-TGT-GGC-GTA-3'
5'-GTT-GGA-GCT-AGT-GGC-GTA-3'
5'-GTT-GGA-GCT-GCT-GGC-GTA-3'
5'-GTT-GGA-GCT-GAT-GGC-GTA-3'
5'-GTT-GGA-GCT-GTT-GGC-GTA-3'

2. Verfahren nach Anspruch 1,
**dadurch gekennzeichnet, daß**
in dem zur Lyse der in der Stuhlprobe vorhandenen Zellen verwendete Puffer die chaotropen Salze Guanidinisothiocyanat, Guanidinhydrochlorid, Lithiumchlorid oder Lithiumchlorid/ Harnstoff-Gemische mit Ionenstärken > 4M Verwendung finden.

3. Verfahren nach einem der Ansprüche 1 oder 2,
**dadurch gekennzeichnet, daß**
als mineralische Trägermaterialien nichtporöse SiO₂- Partikel mit einer Korngröße von 7 nm - 1 µm, vorzugsweise 40 nm, bei einer spezifischen Oberfläche von 10-300 m²/g, vorzugsweise 50 m²/g, eingesetzt werden

4. Verfahren nach einem der Ansprüche 1 bis 3,
**dadurch gekennzeichnet, daß**
die Inkubation der eluierten genomischen DNS mit einem Puffer aus Natriumjodid, Natriumperchlorat oder Kaliumjodid mit Ionenstärken >4M bei kontinuierlichem Schütteln erfolgt.

5. Verfahren nach einem der Ansprüche 1 bis 4,
**dadurch gekennzeichnet, daß**
die Lyse mit einem Puffer enthaltend Guanidinisothiocyanat, DTT und Natriumcitrat und die zweite Inkubation mit 5 M Natriumjodid durchgeführt wird.

6. Verfahren nach einem der Ansprüche 1 bis 5,
**dadurch gekennzeichnet, daß**
die Oligonukleotide und die nachzuweisenden sequenzveränderten Abschnitte des Ki-ras Gens inkubiert werden, wobei entweder die Oligonukleotide oder die nachzuweisenden sequenzveränderten Abschnitte des Ki-ras Gens markiert sind und der Nachweis des Hybridisierungsergebnisses über die Markierung detektiert wird oder andere an sich bekannte Nachweisverfahren für Duplex-DNS zum Nachweis des Hybridisierungsergebnisses verwendet werden.

7. Verfahren nach einem der Ansprüche 1 bis 6,
**dadurch gekennzeichnet, daß**
die Oligonukleotide und/oder die sequenzveränderten Abschnitte des Ki-ras Gens oder hybridisierte DNS sich an einer festen Phase befinden.

8. Verfahren nach Anspruch 7,
**dadurch gekennzeichnet, daß**
die feste Phase eine Mikrotiterplatte, ein Lichtwellenleiter oder ein Siliciumchip ist.

9. Verfahren nach Anspruch 6,
**dadurch gekennzeichnet, daß**
die nachzuweisende Ki-ras-Sequenz mit einem Primerpaar, wobei einer der Primer an seiner 5'-Position markiert ist, amplifiziert wird, anschließend an eine feste Phase gekoppelt und in einen Einzelstrang überführt wird, mit den sechs Oligonukleotiden 15-45 Minuten bei einer Temperatur von 38-45 °C inkubiert, mit einer Waschlösung aus SDS und SSC bei 45-55 °C ein bis fünf mal für 5 bis 15 Minuten gewaschen wird.

10. Verfahren nach Anspruch 9,
**dadurch gekennzeichnet, daß**
die Kopplung über eine Streptavidin-Biotin-Brücke erfolgt, die Inkubation 30 Minuten bei Temperatur von 42 °C stattfindet und 3 mal/10min mit einer Lösung 0,03 % SDS und 0,03 % SSC bei 50 °C gewaschen wird.

11. Verwendung des Verfahrens nach den Ansprüchen 1 bis 10 zur Früherkennung von Tumoren auf der Basis von klinisch relevanten Veränderungen der DNS-Sequenz des Ki-ras Onkogens in DNS, vorzugsweise zur Früherkennung von Pankreas- und Dickdarmtumoren aus Stuhlproben, ercp-Flüssigkeiten und endoskopisch gewonnenen Darmpolypen.

12. Testkit zur Früherkennung von Tumoren auf der Basis von klinisch relevanten Veränderungen der DNS-Sequenz des Ki-ras Onkogens in DNS, vorzugsweise zur Früherkennung von Pankreas-und Dickdarmtumoren aus Stuhlproben umfassend
- DNS-Extraktionssystem enthaltend Lösungen und Puffer gemäß Ansprüchen 1, 2 , 4 bis 6 ,
- Primermixen zur selektiven Amplifikation der zu untersuchenden Ki-ras-Targetsequenz
- Kontrollzellinien-DNS zur Amplifikation von Wildtyp bzw. Mutationstyp einer Ki-ras-Zellinie,
- Oligonukeotidsonden gemäß Anspruch 1 ,
- eine feste Phase gemäß Anspruch 8 samt Reagenzien gemäß Anspruch 9 oder 10.

13. Testkit nach Anspruch 12,
**dadurch gekennzeichnet, daß**
- die feste Phase eine Streptavidin-beschichtete
- Mikrotestplatte ist.

## Claims

1. Procedure to verify clinically relevant changes in the DNA sequence of the Ki-ras oncogene in the DNA with stool probes as source material,
**characterized by**
- extraction of genomic DNA from stool probes via multiple cleansing steps for the elimination of inhibitoric substances with the extraction of the genomic DNA being carried out via:
- removal of inhibitoric substances through incubation with a solution containing active coal,
- lysis of the cells in the stool probe with a buffer solution containing the caotrope salts guanidin isothiocyanate, guanidin hydrochloride, lithium chloride or mixtures of lithium chloride and urea;
- incubation of the lysate with a mineral substrate made of non-porous particles of SiO₂ for bonding the DNA;
- Seperation of the substrate from the lysate via centrifugation;
- washing of the DNA bonded to the substrate with a mixture of 50 mM NaCl, 10 mM Tris HCl and 1 mM EDTA as well as 70%v/v ethanol and elution of the DNA via incubation of the substrate with a buffer solution of a low ionic strength made of 10 mM Tris HCI, 0,1 mM EDTA at a temperature of 48 - 65°C;
- incubation of the unsoldered genomic DNA with a buffer solution made of sodium iodide, sodium perchlorate or potassium iodide for eliminating substances bonded to the DNA from the genomic DNA;
- renewed incubation of the buffer solution with the genomic DNA by means of a mineral substrate;
- separation of the solution from the mineral substrate via centrifugation;
- washing of the substrate with the bonded DNA and renewed elution of the bonded genomic DNA with a buffer solution of a low ionic strength
and
- hybridisation reaction of complementary bases by adding the following six oligonucleotides with a defined complementarity to changed sequence segments of the Ki-ras gene:
5'-GTT-GGA-GCT-CGT-GGC-GTA-3'
5'-GTT-GGA-GCT-TGT-GGC-GTA-3'
5'-GTT-GGA-GCT-AGT-GGC-GTA-3'
5'-GTT-GGA-GCT-GCT-GGC-GTA-3'
5'-GTT-GGA-GCT-GAT-GGC-GTA-3'
5'-GTT-GGA-GCT-GTT-GGC-GTA-3'

2. Procedure according to claim 1,
**characterized by** the fact that
the caotrope salts guanidin isothiocyanate, guanidin hydrochloride, lithium chloride or mixtures of lithium chloride and urea with ionic strenghts > 4M are utilised in buffer solutions which are used for the lysis of the cells in the stool probe.

3. Procedure according to one of the claims 1 to 2,
**characterized by** the fact that
non-purous SiO₂ particles with a particle size of 7 nm - 1 µm, preferably 40 nm with a specific surface of 10 - 300 m²/g, preferably 50 m²/g are used as mineral substrates.

4. Procedure according to one of the claims 1 to 3,
**characterized by** the fact that
the incubation of the eluted genomic DNA is carried out with a buffer solution made of sodium iodide, sodium perchlorate or potassium iodide with ionic strengths > 4M while continuously shaking.

5. Procedure according to one of the claims 1 to 4
**characterized by** the fact that
the lysis is carried out with a buffer solution containing guanidin isothiocyanate, DTT and sodium citrate and the second incubation with 5 M sodium iodide.

6. Procedure according to one of the claims 1 to 5,
**characterized by** the fact that
the oligonucleotides and the sections of the Ki-ras gene changing the DNA which have to be verified with either the oligonucleotides or the sections of the Ki-ras gene changing the DNA which have to be verified being marked and the verification of the result of the hybridisation is detected by means of the marking or other verification procedures for Duplex DNA known in principle are used for verifying the result of the hybridisation.

7. Procedure according to one of the claims 1 to 6,
**characterized by** the fact that
the oligonucleotides and / or the sections of the Ki-ras gene changing the DNA or the hybridised DNA are in a solid phase.

8. Procedure according to claim 7,
**characterized by** the fact that
the solid phase is a micro titre plate, a light wave guide or a silicon chip.

9. Procedure according to claim 6,
**characterized by** the fact that
the sequence of the Ki-ras gene which has to be verified is amplified with a pair of primers with one primer being marked on its 5' position, is subsequently coupled to a solid phase and transferred into its single strand, is incubated with the six oligonucleotides for 15 - 45 minutes at a temperature of 38 - 45 °C and is washed with a washing solution made of SDS and SSC at a temperature of 45 - 55°C one to five times for 5 to 15 minutes.

10. Procedure according to claim 9,
**characterized by** the fact that
the linkage is carried out by means of a link between steptavidin and biotin, the incubation takes place for 30 minutes at a temperature of 42°C and it is being washed for three times 10 minutes each with a solution of 0,03 % SDS and 0,03 % SSC at 50°C.

11. Application of the procedure according to the claims 1 to 10 for the early diagnosis of tumours on the basis of clinically relevant changes of the sequence of the DNA of the Ki-ras oncogene in the DNA, preferably for the early diagnosis of pancreatic and colon tumours from stool probes, ercp liquids and intestinal polyps obtained by means of endoscopy.

12. Testkit for the early diagnosis of tumours on the basis of clinically relevant changes of the sequence of the DNA of the Ki-ras oncogene in the DNA preferably for the early diagnosis of pancreatic and colon tumours from stool probes, including
- system for DNA extraction containing solutions and buffer solutions according to the claims 1, 2 , 4 to 6 ,
- mixtures of primers for the selective amplification of the Ki-ras target sequence which has to be analysed
- DNA of control cell lines for the amplification of the wild type resp. mutation type of one Ki-ras cell line,
- probes of oligonucleotides according to claim 1,
- a solid phase according to claim 8 including the reagents according to claim 9 or 10.

13. Testkit according to claim 12,
**characterized by** the fact that
- the solid phase is a microtest plate laminated with streptavidin.

## Revendications

1. Procédé de mise en évidence de modifications cliniques importantes de la séquence d'ADN de l'oncogène Ki-ras dans l'ADN, des échantillons de selles servant de matériel de départ,
**se caractérisant par le fait que**
- Extraction de l'ADN génomique d'échantillons de selles par l'intermédiaire de multiples étapes de purification pour éliminer les substances inhibitrices, l'extraction de l'ADN génomique se faisant par :
- Élimination de substances inhibitrices par incubation avec une solution contenant du charbon actif,
- Lise des cellules contenues dans l'échantillon de selles avec un tampon qui contient les sels chaotropes de sothiocyanate de guanidine, hydrochlorure de guanidine, chlorure de lithium ou mélange de chlorure de lithium/urée ;
- Incubation du lysat avec un matériau support minéral composé de particules de SiO₂ non poreuses qui lient l'ADN.
- Séparation du matériau support du lysat par centrifugation ;
- Lavage de l'ADN lié au matériau support avec un mélange de 50 mM NaCl, 10 mM Tris HCl et 1 mM EDTA ainsi que 70 % (v/v) d'éthanol et élution de l'ADN par incubation du matériau support avec un tampon de force ionique faible composé de 10 mM Tris HCl, 0,1 mM EDTA pour une température de 48-65 °C ;
- Incubation de l'ADN génomique détaché avec un tampon composé d'iodure de sodium, de perchlorate de sodium ou d'iodure de potassium pour éliminer les substances liées à l'ADN de l'ADN génomique ;
- Nouvelle incubation de la solution tampon contenant l'ADN génomique avec un matériau support minéral ;
- Séparation de la solution du matériau support minéral par centrifugation ;
- Lavage du matériau support contenant l'ADN lié et nouvelle élution de l'ADN génomique lié avec un tampon de force ionique faible
et
- Réaction d'hybridation à complémentarité de bases par l'addition des six oligonucléotides suivants avec une complémentarité définie aux sections de séquence modifiées du gène Ki-ras :
5'-GTT-GGA-GCT-CGT-GGC-GTA-3'
5'-GTT-GGA-GCT-TGT-GGC-GTA-3'
5'-GTT-GGA-GCT-AGT-G.GC-GTA-3'
5'-GTT-GGA-GCT-GCT-GGC-GTA-3'
5'-GTT-GGA-GCT-GAT-GGC-GTA-3'
5'-GTT-GGA-GCT-GTT-GGC-GTA-3'

2. Procédé selon la revendication 1,
**se caractérisant par le fait que**
dans le tampon utilisé pour la lyse des cellules existant dans l'échantillon de selles, les sels chaotropes de sothiocyanate de guanidine, hydrochlorure de guanidine, chlorure de lithium ou mélange de chlorure de lithium/urée avec des forces ioniques > 4M sont employés.

3. Procédé selon l'une des revendications 1 ou 2,
**se caractérisant par le fait que**
des particules SiO₂ non poreuses avec une grosseur de grain entre 7 nm et 1 µm, de préférence 40 nm, pour une surface spécifique de 10 à 300 m²/g, de préférence 50 m²/g, sont utilisées en tant que matériau support minéral

4. Procédé selon l'une des revendications 1 à 3,
**se caractérisant par le fait que**
l'incubation de l'ADN génomique élué avec un tampon composé d'iodure de sodium, de perchlorate de sodium ou d'iodure de potassium avec des forces ioniques >4M se fait sous l'effet de secousses continues.

5. Procédé selon l'une des revendications 1 à 4,
**se caractérisant par le fait que**
la lyse se fait avec un tampon contenant du sothiocyanate de guanidine, DTT et du citrate de sodium et la deuxième incubation avec 5 M de iodure de sodium.

6. Procédé selon l'une des revendications 1 à 5,
**se caractérisant par le fait que**
les oligonucléotides et les sections à séquence modifiée à mettre en évidence du gène Ki-ras sont incubés, les oligonucléotides ou les sections à séquence modifiée à mettre en évidence étant marqués et la mise en évidence du résultat de l'hybridation étant détectée par le marquage ou d'autres procédés de mise en évidence connus pour l'ADN duplex étant utilisés pour mettre en évidence le résultat de l'hybridation.

7. Procédé selon l'une des revendications 1 à 6,
**se caractérisant par le fait que**
les oligonucléotides et/ou les sections à séquence modifiée du gène Ki-ras ou de l'ADN hybridé se trouvent en phase solide.

8. Procédé selon la revendication 7,
**se caractérisant par le fait que**
la phase solide est une plaque microtitre, un guide d'ondes lumineuses ou une micropastille de silicium.

9. Procédé selon la revendication 6,
**se caractérisant par le fait que**
la séquence Ki-ras à mettre en évidence avec une paire d'amorces, l'une des amorces étant marquée à sa position 5', est amplifiée, ensuite couplée à une phase solide et transformée en un brin simple, incubé avec les six oligonucléotides durant 15-45 minutes à une température de 38-45 °C, lavée avec une solution de lavage composée de SDS et SSC à 45-55 °C une à cinq fois durant 5 à 15 minutes.

10. Procédé selon la revendication 9,
**se caractérisant par le fait que**
le couplage se fait via un pont de streptavidine-biotine, l'incubation a lieu 30 minutes à une température de 42 °C et est lavée 3 fois/10 mn avec une solution de 0,03 % de SDS et de 0,03 % de SSC à 50 °C.

11. Emploi du procédé selon les revendications 1 à 10 pour le dépistage précoce de tumeurs sur la base de modifications cliniques importantes de la séquence d'ADN de l'oncogène Kiras dans l'ADN, de préférence pour le dépistage précoce de tumeurs du pancréas et du colon à partir d'échantillons de selles, de liquides de l'ERCP et de polypes intestinaux prélevés par endoscopie.

12. Kit test pour le dépistage précoce de tumeurs sur la base de modifications cliniques importantes de la séquence d'ADN de l'oncogène Ki-ras dans l'ADN, de préférence pour le dépistage précoce de tumeurs du pancréas et du colon à partir d'échantillons de selles, comprenant
- un système d'extraction de l'ADN contenant des solutions et des tampons selon les revendications 1, 2, 4 à 6,
- des mélanges d'amorces pour l'amplification sélective de la séquence cible de Ki-ras à analyser
- ADN de lignes cellulaires de contrôle pour l'amplification du type sauvage ou mutant d'une ligne cellulaire Ki-ras,
- des sondes d'oligonucléotides selon la revendication 1,
- une phase solide selon la revendication 8 avec les réactifs conformément à la revendication 9 ou 10.

13. Kit test selon la revendication 12
**se caractérisant par le fait que**
- la phase solide est une plaque microtest recouverte de streptavidine.
